# EUROPÄISCHE PATENTSCHRIFT

(11) **EP 3 440 099 B1**
(45) Veröffentlichungstag und Bekanntmachung des Hinweises auf die Patenterteilung: **17.06.2020**
(21) Anmeldenummer: 17724313.6
(22) Anmeldetag: 04.04.2017
(51) Int. Cl.: C07K 14/435

(54) **POLYMERBASIERTES MATERIAL MIT KOVALENT GEBUNDENEN, ENZYMATISCH ABBAUBAREN PEPTIDSEQUENZEN**
POLYMER BASED MATERIAL WITH COVALENTLY BONDED, ENZYMATICALLY DEGRADABLE PEPTIDE SEQUENCES
MATERIAU POLYMERISABLE COMPRENANT DES SEQUENCES PEPTIDIQUES DEGRADABLES PAR ENZYMES, LIEES DE MANIERE COVALENTE

(30) Priorität: 04.04.2016 EP 16163667
(43) Veröffentlichungstag der Anmeldung: 13.02.2019
(73) Patentinhaber: TissueGUARD GmbH, 01217 Dresden (DE)
(72) Erfinder: TSURKAN, Mikhail, 01217 Dresden (DE); BESSERT, Juliane, 42659 Solingen (DE); WERNER, Carsten, 01324 Dresden (DE)
(74) Vertreter: Lippert Stachow Patentanwälte Rechtsanwälte Partnerschaft mbB
(86) Internationale Anmeldenummer: PCT/DE2017/100260
(87) Internationale Veröffentlichungsnummer: WO 2017/174071

(56) Entgegenhaltungen:
- EP-A1- 2 511 289
- WO-A1-2013/071126
- WO-A1-2014/039245
- WO-A2-02/50242
- MIKHAIL V. TSURKAN ET AL: "Defined Polymer-Peptide Conjugates to Form Cell-Instructive starPEG-Heparin Matrices In Situ", ADVANCED MATERIALS, Bd. 25, Nr. 18, 14. Mai 2013 (2013-05-14), Seiten 2606-2610, XP055393137, DE ISSN: 0935-9648, DOI: 10.1002/adma.201300691

## Beschreibung

Die Erfindung betrifft ein polymerbasiertes Material mit kovalent gebundenen, enzymatisch abbaubaren Peptidsequenzen. Des Weiteren betrifft die Erfindung die Verwendung eines solchen polymerbasierten Materials für eine *in-vitro-*Herstellung von Zellkulturen oder Geweben oder Organen, für eine *in-vitro-*Stabilisierung von gespendeten Zellen, Geweben oder Organen sowie für eine *in-vivo-* oder *in-vitro-*Behandlung von lebenden Zellen, Geweben und Organen. Dabei ist eine Haftbindung zwischen dem Material einerseits und der Probe andererseits, das heißt Zellen, Geweben oder Organen, gesteuert abbaubar, ohne die Integrität der Probe (extrazelluläre Matrix sowie Zell-Zell und extrazelluläre Matrix-Zell Kontakte) zu zerstören (Bioorthogonalität).

Es gibt eine Reihe von Techniken für die Bildung von zellkompatiblen Materialien und für deren individuelle Funktionalisierung mit einer Vielzahl von biologisch aktiven Molekülen, wie kovalent gebundenen zelladhäsiven Peptiden, Proteinen und teilweise von Wachstumsfaktoren und Zytokinen. Im Vergleich zu solchen Verfahren sind synthetische Methoden zur Einführung von selektiven Materialzersetzungsreaktionen im Wesentlichen unerforscht. Die bisher angewendeten enzymatischen Verfahren zur Zellablösung/Dissoziation, wie Verfahren unter Anwendung von Trypsin, Pepsin oder Collagenasen, sind weder für neu erzeugte noch für gespendete Gewebe und Organe anwendbar, da diese unselektiven Verfahren die Ordnung von deren extrazellulärer Matrix beschädigen. Darüber hinaus ist die Anwendung derartiger Enzyme zur Zellfreisetzung in dreidimensionalen Materialien stark begrenzt, da dies eine höhere Enzymkonzentration oder längere Behandlungszeiten benötigt, die zur unerwünschten Spaltung der Zelloberflächen-Proteine führen kann. Selektivere Matrix-Metalloproteasen (MMPs), die bestimmte Aminosäuresequenzen spalten, haben sich als für die schonende Zellentnahme in dreidimensionalen Hydrogelmaterialien anwendbar gezeigt. Die am häufigsten verwendeten MMPs haben eine geringe Wirkung auf das Zellverhalten gezeigt. Dennoch ist die Verwendung von MMPs im Allgemeinen auf Gewebe- oder Organ-Anwendungen nur eingeschränkt möglich, da sie eine Spaltung von Proteinen der Kollagenfamilie in der extrazellulären Matrix (EZM) hervorrufen, was zum Verlust wichtiger Struktureinheiten der Gewebe und Organe führen kann. Zusammengefasst bietet der aktuelle Stand der Technik praktische Techniken zur Zellentnahme sowohl in zwei- als auch in dreidimensionalen Anwendungen, während bis jetzt keine geeigneten biotechnologischen Verfahren für die schonende Gewinnung von komplexen Zellorganisationen wie Geweben und Organen aus Trägermaterialien zur Verfügung stehen.

Aus der WO 02/50242 A2 ist ein Verfahren zum In-vitro-Wachstum von Gewebe bekannt. Dabei wird ein verformbares Substrat verwendet, welches mit gewebebildenden Zellen besiedelt ist und welches mindestens einen Strömungskanal definiert, der ein flüssiges Kulturmedium enthält. Das Substrat kann aus einem Material wie einem synthetischen Polymer oder einem aggregierten natürlichen Makromolekül, beispielsweise einem Protein oder Polysaccharid, hergestellt sein. Das Substrat kann abbaubar ausgebildet sein, indem es eingebaute Peptidsequenzen aufweist, die durch Proteasen spaltbar sind.

In der WO 2013/071126 A1 ist ein Verfahren zur Herstellung eines Hydrogels mit einer räumlich kontrollierten dreidimensionale Verteilung eines oder mehrerer bioaktiver Signale beschrieben. Das Hydrogel enthält dabei ein Polymer, welches gekoppelt ist an ein Peptid mit einer Aminosäure mit photolabiler Schutzgruppe. Diese Schutzgruppe ist so positioniert, dass sie einen enzymatischen Abbau der Protease-spaltbaren Sequenz des Peptids verhindert. Erst nach lichtinduzierter Abspaltung der Schutzgruppe wird ein durch Protease abbaubares Peptid generiert, welches mindestens eine durch eine Protease, zum Beispiel MMPs, spaltbare Stelle aufweist.

Aus der WO 2014/039245 A1 ist ein Verfahren zur Herstellung eines Hydrogels bekannt, welches die Kondensation von zwei funktionellen Gruppen umfasst, wobei die erste Gruppe ein Molekül oder Makromolekül enthält, welches zwei oder mehr Hydroxylamingruppen oder Aminooxygruppen aufweist, und die zweite Gruppe ein Molekül oder Makromolekül enthält, welches zwei oder mehr Aldehyd-, Keton- oder andere reaktive Oxogruppen aufweist. Die Kondensation findet unter solchen Bedingungen statt, unter denen sich ein Hydrogel bildet. Dabei kann das Hydrogel eine enzymatisch spaltbare Gruppe, zum Beispiel eine MMP-spaltbare Gruppe enthalten, die zwischen einem Polypeptid und einem Makromolekül platziert ist. Bei Enzymzugabe wird dann das Polypeptid freigesetzt.

Aus der Druckschrift MIKHAIL V. TSURKAN ET AL: "Defined Polymer-Peptide Conjugates to Form Cell-Instructive starPEG-Heparin Matrices In Situ**"** sind Polymer-Peptid-Konjugate zur Bildung von zellinstruktiven Matrices aus Stern-Polyethylenglykol und Heparin bekannt. Auch hier werden Hydrogele erhalten, die mittels MMP spaltbar sind.

Die EP 2 511 289 A1 betrifft ein Verfahren zur Herstellung eines glykosylierten Peptids, umfassend die Bereitstellung eines Peptids, das mit einem oder mehreren hydrophilen Polymeren konjugiert ist, und die Glykosylierung des Polymer-konjugierten Peptids mittels einer oder mehrerer Glycosyltransferasen. Nach Isolierung der glykosylierten Konjugate aus der Glykosylierungsreaktion folgt die Freisetzung der Glykopeptidprodukte aus den mit den Polymer-konjugierten Vorprodukten mittels einer Protease. Dafür ist eine proteolytisch spaltbare Stelle vorgesehen, beispielsweise eine Spaltungsstelle der Tabakätzvirus (TEV) -Protease, der Faktor Xa-Protease, der SUMO-Protease, der Tabakvenen-Sprenkelvirus (TVMV)-Protease oder eine 3C-Protease, wobei die spaltbare Stelle zum Beispiel zwischen dem hydrophilen Polymer und der Glykosylierungsstelle lokalisiert sein kann.

Neben den klassischen enzymatischen Methoden gibt es einige für die Zukunft vielversprechende Techniken der Zellentnahme, die auf der Beeinflussung der physikochemischen Eigenschaften der verwendeten Materialien basieren. Zum Beispiel wurden einige Fortschritte bei der Entwicklung von thermoresponsiven Bio-Hybrid-Materialien erreicht, die eine mechanische Spannung bei Temperaturänderungen induzieren. Eine solche Belastung, beispielsweise das Aufquellen einer Hydrogelstruktur, induziert die Ablösung (Delaminierung) von anhaftenden Zellen, und wurde daher für die Herstellungen von einlagigen Zellschichten verwendet, für das sogenannte Cell-Sheet-Engineering. Dennoch erfordert dieses Verfahren, die Zellhaftung und die induzierte Ablösung im Gleichgewicht zu halten und benutzerdefinierte Trägereigenschaften zu erzeugen, um für verschiedene Zelltypen einsetzbar zu sein. Ohne eine solche zeit- und ressourcenintensive Erzeugung/Anpassung kann die Materialinduzierte Belastung die zelluläre EZM beschädigen, die an die Materialien angebracht ist, wodurch die Reproduzierbarkeit solcher Verfahren begrenzt ist.

Außerdem ist dieser Stand der Technik ausschließlich auf einfache zweidimensionale Anwendungen beschränkt und eine Eignung der temperaturempfindlichen Träger für mehrschichtige oder komplexere Gewebe ist fraglich. Eine solche Begrenzung der Einsetzbarkeit wird stärker, wenn es zum Transport oder zur Behandlung von Organen kommt: Thermoresponsive Träger sind dann nicht mehr anwendbar, da sie keine ausreichend starke Haftung zulassen würden (die Polymerschichten sind nur einige hundert Nanometer dick). Ein weiterer Nachteil der thermoresponsiven Träger ist die für die Delaminierung benötigte Temperaturänderung: Es gibt Zellen, die sehr empfindlich sind und langfristige Temperaturänderungen/Temperaturabfälle nicht unbeschadet überstehen.

In jüngster Zeit führte das wachsende Interesse in Richtung orthogonaler Methoden in der Materialchemie zur Erzeugung von Hydrogelen, die orthogonal durch Licht und Zellen gespalten werden könnten. Die Verwendung von Zwei-Photonen-Techniken erlaubt die Anwendung solcher Verfahren für eine lichtgesteuerte Zellflucht/Abspaltung aus Hydrogel-Gerüsten. Mit einigen Anpassungen könnten solche Systeme für die Zellentnahme eingesetzt werden, aber die derzeitige Begrenzung auf Zwei-Photonen-Geräte und die geringe Biokompatibilität der entsprechenden Photonen-Reaktionen beschränken diese vielversprechende Technik auf Einzelzellanwendungen.

Zusammengefasst verfügen die derzeit am besten entwickelten physikalischchemischen Methoden zur Materialbeeinflussung nicht die notwendige Instrumentierung für die schonende Zellabspaltung aus Trägermaterialien von komplexen Zellorganisationen wie Geweben und Organen.

Die Entwicklung kompatibler Materialien für die De-novo-Gewebe-Erzeugung, das Gewebe-Engineering oder zur Stabilisierung von gespendeten Geweben oder Organ-Implantaten ist ein allgemeiner Ansatz in den Bereichen der regenerativen Medizin und Implantatforschung. Die Fortschritte in diesen Forschungsgebieten führten zu dem heutigen Verständnis über die Bedeutung von Selbstregenerationsmethoden, in denen *ex vivo* Gewebe oder Organe aus wenigen Original-Zellen neu erzeugt werden können. Diese Methoden bringen verschiedene neue Probleme - zum Teil auch Selbstausschluss-Probleme - in die Materialentwicklung für die Gewebe- und Organimplantat-Herstellung. Einerseits erfordern Zellkulturen stabile biofunktionalisierte Oberflächen oder Gerüste, die der dreidimensionalen extrazellulären Matrix ähnlich sind und welche für die Expansion der Zellen und eine anschließende Gewebeerzeugung durch die Zelle abbaubar sind. Starke zellhaftende Eigenschaften solcher Materialien sind ein wichtiges Merkmal für eine erfolgreiche Zell- oder Gewebeentwicklung. Andererseits sollte für eine erfolgreiche Implantation das gebildete Gewebe schonend vom Trägermaterial abgetrennt werden, um dessen zellulären und extrazellulären Matrix-Aufbau unberührt zu erhalten. Mit anderen Worten, es sollte die Haftverbindung zwischen Material und Gewebe abgebaut werden, ohne die extrazelluläre Matrix sowie Zell-Zell und extrazelluläre Matrix-Zell Bindungen innerhalb des Gewebes zu zerstören. Da aber diese Bindungen gleicher oder ähnlicher chemischer Natur sind, erfordert ihre selektive Spaltung neuartige, nicht triviale bioorthogonale Methoden zur selektiven Zersetzung der Haftverbindung zwischen Material und Gewebe.

Die der Erfindung zugrundeliegende Aufgabe besteht daher darin, ein Material bereitzustellen, bei dem eine Haftbindung zwischen dem Material und Zellen, Geweben oder Organen gesteuert und selektiv abbaubar ist, ohne die Bindung zwischen den Zellen und der extrazellulären Matrix innerhalb des Gewebes zu zerstören.

Die Aufgabe der Erfindung wird durch ein polymerbasiertes Material mit kovalent gebundenen, enzymatisch abbaubaren Peptidsequenzen nach Anspruch 1 gelöst. Dabei sind die abbaubaren, vorzugsweise spaltbaren Peptidsequenzen gegenüber der allgemeinen biologischen und metabolischen Aktivität von Zellen und Geweben inert, das heißt, sie sind durch die biologische und metabolische Aktivität von Zellen und Geweben nicht abbaubar. Die Peptidsequenzen bestehen jeweils aus zwei bis fünfzehn Aminosäuren und können in das polymerbasierte Material eingebaut oder an das polymerbasierte Material konjugiert werden. Somit kann die Peptidsequenz entweder Teil einer drei- oder zweidimensionalen Struktur des polymerbasierten Materials sein. Mittels eines Enzym-Zugabe gesteuerten Abbaus einer kovalenten Bindung der Peptidsequenz erfolgt entweder ein bioorthogonaler Abbau der dreidimensionalen Struktur, oder im Falle einer Konjugation der Peptidsequenz an das polymerbasierte Material die Freisetzung zumindest eines Teils des Moleküls.

Vorzugsweise beträgt die Zahl der Aminosäurereste pro Peptidmolekül 5 bis 10. Das polymerbasierte Material kann eine zwei oder dreidimensionale Netzwerkstruktur mit Knoten und Kanten bilden, wobei zumindest ein Teil der Knoten und/oder Kanten die Verbindungsmoleküle enthalten. Das polymerbasierte Material kann zum Beispiel ein Hydrogel oder ein vorzugsweise festes kondensiertes Polymer sein. Gemäß einer vorteilhaften Ausführungsform der Erfindung kann das polymerbasierte Material eine Polymerbeschichtung eines Trägersubstrates (zum Beispiel Glas) sein.

Nach einer besonders bevorzugten Ausgestaltung der Erfindung umfasst das polymerbasierte Material mindestens eine vorzugsweise bioaktive Komponente, die durch einen mittels Enzym-Zugabe gesteuerten Abbau der kovalenten Bindung der Peptidsequenz freisetzbar ist. Diese Komponente gehört einer Gruppe von Komponenten an, bestehend aus Wirkstoffen, Nukleinsäuren, zum Beispiel DNA, RNA oder Aptameren, Proteinen, Peptid-Konjugaten, sulfatierten und nicht sulfatierten Polysacchariden und deren Konjugaten. Das Protein beziehungsweise Peptid-Konjugat kann dabei vorteilhafterweise ein Zytokin, ein Chemokin, ein Wachstumsfaktor, ein Hormon, Antikörper oder eine Komponente der extrazellulären Matrix sein.

Die Moleküle der mindestens einen freisetzbaren Komponente sind entsprechend einer Ausführungsvariante durch Linker, die jeweils die enzymatisch abbaubare Peptidsequenz enthalten, an das polymerbasierte Material konjugiert.

Alternativ können die Moleküle der mindestens einen freisetzbaren Komponente in ein dreidimensionales Polymernetzwerk physikalisch eingeschlossen und daran physikalisch gebunden sein. Durch einen bioorthogonalen Abbau des einschließenden Polymernetzwerkes würden die Komponenten dann freigesetzt werden.

Gemäß der Erfindung sind die Peptidsequenzen enzymatisch abbaubar, indem sie eine durch Proteasen enzymatisch spaltbare Stelle aufweisen. Auch die Freisetzung von Wirkstoffen, Nukleinsäuren, Proteinen, Peptid-Konjugaten oder Polysacchariden kann dann über die Protease-Zugabe gesteuert werden.

Erfindungsgemäß weist die spaltbare Peptidsequenz dabei eine spaltbare Stelle auf, ausgewählt aus einer Gruppe von Spaltungsstellen, bestehend aus einer Gruppe von spaltbaren Stellen, bestehend aus einer durch Tabakätzvirus-Protease spaltbaren Stelle, einer durch humane Rhinovirus-3C-Protease spaltbaren Stelle, einer durch Faktor-Xₐ-Protease spaltbaren Stelle, einer durch Thrombin-Protease spaltbaren Stelle, einer durch Enterokinase spaltbaren Stelle, einer durch Sortase-A-Protease spaltbaren Stelle, einer durch Caspase-3-Protease spaltbaren Stelle, einer durch Granzym-B-Serin-Protease spaltbaren Stelle sowie einer durch PreScission™ spaltbaren Stelle. PreScission™ ist ein Fusionsprotein aus Glutathion-S-Transferase (GST) und Humaner-Rhinovirus(HRV)-Typ-14-3C-Protease.

Als enzymatisch abbaubaren Peptidsequenzen werden vorteilhafterweise solche Peptidsequenzen ausgewählt, die durch die biologische und metabolische Aktivität von Zellen und Geweben nicht abbaubar sind. Die Peptidsequenz wird dabei vorzugsweise aus einer Gruppe ausgewählt, die aus ENLYFQ/X, ETVLFQ/GP, EVLFQ/GP, IEGR/IEGRX, CWGGGIEGR/MGGCG, CGGGIEGR/MGGWCG, DDDDK/, LVPRGS/FXRS, DXXD/, LEVLFQ/GP, LPET/G, DX, NX, MN und SX besteht, wobei der Schrägstrich (/) jeweils die spaltbare Stelle anzeigt, die Buchstaben den Einbuchstabencode der proteinogenen Aminosäuren und X eine beliebige natürlich vorkommende Aminosäure darstellen.

Gemäß einer weiteren Ausgestaltung der Erfindung umfasst das polymerbasierte Material ferner Peptidsequenzen, die durch die biologische und metabolische Aktivität von Zellen und Geweben abbaubar sind, und derart in das polymerbasierte Material eingebaut oder an das polymerbasierte Material konjugiert sind, dass die Peptidsequenz entweder Teil einer zwei- oder dreidimensionalen Struktur des polymerbasierten Materials ist. Durch die biologische und metabolische Aktivität von Zellen und Geweben erfolgt ein bioorthogonaler Abbau der dreidimensionalen Struktur, oder im Falle einer Konjugation der Peptidsequenz an das polymerbasierte Material die Freisetzung zumindest eines Teils des Moleküls. Diese Peptidsequenzen sind vorzugsweise mit den durch die biologische und metabolische Aktivität von Zellen und Geweben nicht abbaubaren Peptidsequenzen verbunden. Eine gesteuerte Zugabe eines Enzyms, das heißt einer Zugabe des Enzyms bei Bedarf, führt hier zu einer Beschleunigung des Abbaus des Materials beziehungsweise zu einer beschleunigten Freisetzung von Komponenten.

Die vorliegende Erfindung kann in Verfahren zur bedarfsweisen, das heißt zeitlich gesteuerten Spaltung einer kovalenten Bindung in polymerbasierten Materialien verwendet werden, wobei diese Verfahren in Gegenwart von lebenden Zellen, Geweben oder Organen *in vitro* oder *in vivo* bioorthogonal ausgeführt werden können. Diese Verwendungen/Verfahren basieren auf dem Einbau der kurzen enzymatisch abbaubaren/spaltbaren Peptidsequenzen als molekulare Linker in gebräuchliche polymerbasierte Materialstrukturen. Der enzymatisch gesteuerte Abbau der kovalenten Bindung führt zu einem MaterialAbbau, wenn sich die abbaubare kovalente Bindung innerhalb der Materialstruktur befindet. Der enzymatisch gesteuerte Abbau der kovalenten Bindung führt dazu, dass Moleküle freigesetzt werden, wenn die abbaubare Bindung jeweils ein Teil von Molekülen ist, die an das Material konjugiert sind. Die durch die enzymatische Reaktion abbaubaren Bindungen des Materials ermöglichen eine orthogonale Anwendung der enzymatischen Reaktion in Gegenwart lebender Materie, wie Zellen, Geweben oder Organen, und/oder in Gegenwart von Molekülen der extrazellulären Matrix.

Das mit der abbaubaren Peptidsequenz versehene polymerbasierte Material kann für eine gesteuerte *in-vivo-* oder *in-vitro*-Freisetzung eines Wirkstoffs oder einer anderen der oben genannten freisetzbaren bioaktiven Komponenten verwendet werden, wobei die Freisetzung durch einen mittels Enzym-Zugabe gesteuerten Abbau der kovalenten Bindung der Peptidsequenz erfolgt. Eine solche Verwendung kann insbesondere im Rahmen einer Zell-, Gewebe- und Organbehandlung *in vivo* oder *in vitro* erfolgen, bei der bei Bedarf ein Wirkstoff und/oder eine andere der genannten freisetzbaren bioaktiven Komponenten durch Enzymzugabe gesteuert freigesetzt wird/werden.

Darüber hinaus ist das erfindungsgemäße polymerbasierten Materials für eine *in-vitro*-Zellkultur oder *in-vitro*-Herstellung von Gewebe- oder Organen geeignet. Die enzymatische Spaltung des Materials beeinträchtigt dabei nicht die extrazelluläre Matrix sowie Zell-Zell und extrazelluläre Matrix-Zell-Kontakte. Somit ermöglicht es die zerstörungsfreie Trennung einer lebenden Probe, das heißt einer Zelle, Zellschicht eines Gewebes oder Organs, und ihrer extrazellulären Matrix aus dem Trägermaterial durch Materialabbau nach Bedarf zu einem beliebigen Zeitpunkt der Entwicklung der lebenden Probe.

Das mit der abbaubaren Peptidsequenz versehene polymerbasierte Material kann auch zur *in-vitro*-Stabilisierung von gespendeten Zellen sowie von gespendeten Geweben oder Organen verwendet werden. Auch in diesem Fall beeinträchtigt die enzymatische Spaltung des Materials nicht die extrazelluläre Matrix sowie Zell-Zell und extrazelluläre Matrix-Zell-Kontakte. Durch die gesteuerte enzymatische Spaltung der Peptidsequenzen ist es möglich, die Zellen/das Gewebe/das Organ schonend/zerstörungsfrei vom Trägermaterial abzutrennen, um eine erfolgreiche Implantation zu ermöglichen.

Zusammengefasst lässt sich sagen, dass die Verwendung des beschriebenen enzymatischen Abbaus als bioorthogonale Methode für den Materialabbau völlig neue Perspektiven für die Biomaterial-Anwendungen von lebenden Zellen, Geweben und Organen *in vitro* oder *in vivo* eröffnet.

Weitere Einzelheiten, Merkmale und Vorteile von Ausgestaltungen der Erfindung ergeben sich aus der nachfolgenden Beschreibung von Ausführungsbeispielen mit Bezugnahme auf die zugehörigen Zeichnungen. Es zeigen:
- **Fig. 1:**: eine schematische Darstellung von polymerbasierten Materialstrukturen mit eingebauten kurzen enzymatisch spaltbaren Peptidsequenzen als molekulare Linker,
- **Fig.2:**: Beispiele für mögliche funktionellen Oberflächenbeschichtungen mit kurzen enzymatisch spaltbaren Peptidsequenzen als molekulare Linker,
- **Fig. 3:**: Beispiele für einen enzymatisch spaltbaren Peptid-Linker auch als Teil eines Hydrogels oder eines kondensierten polymeren Materials,
- **Fig.4:**: die Verwendung von Wirkstoffen, die an das Material über Peptid-Linker gebunden werden können,
- **Fig.5:**: eine schematische Darstellung der Verwendung eines bioorthogonalen Materialabbaus für die Herstellung von Zell-, Gewebe- Einzelschichten oder Transplantaten,
- **Fig.6:**: eine schematische Darstellung der Anwendung des bioorthogonalen Materialabbaus für die Zell- und Gewebeherstellung in dreidimensionalen Anwendungen,
- **Fig. 7A:**: ein Reaktionsschema einer PEG-Peptid-Konjugatbildung,
- **Fig. 7B:**: eine HPLC-Analyse von PEG-(Maleimid)₄,
- **Fig.7C:**: eine HPLC-Analyse einer Reaktionsmischung von PEG-(Maleimid)₄ mit einem spaltbaren Peptid,
- **Fig. 8A-8B:**: eine schematische Ansicht der Hydrogel-Netzwerkbildung durch eine Michael-Addition,
- **Fig. 9:**: mikroskopische Aufnahmen mit einem Beispiel für eine Langzeit-Kultur auf FXa-abbaubaren Hydrogelen,
- **Fig. 10:**: lichtmikroskopische Aufnahmen der Bildung von Schichten von Humanen cornealen Endothelzellen (HCEC) bei gleichzeitigem Hydrogelabbau,
- **Fig. 11A:**: lichtmikroskopische Aufnahmen der Bildung von Schichten von Humanen cornealen Endothelzellen (HCEC) bei gleichzeitigem Hydrogelabbau und
- **Fig. 11B:**: lichtmikroskopische Aufnahmen verschiedener Beispiele für gebildete HCEC-Schichten.

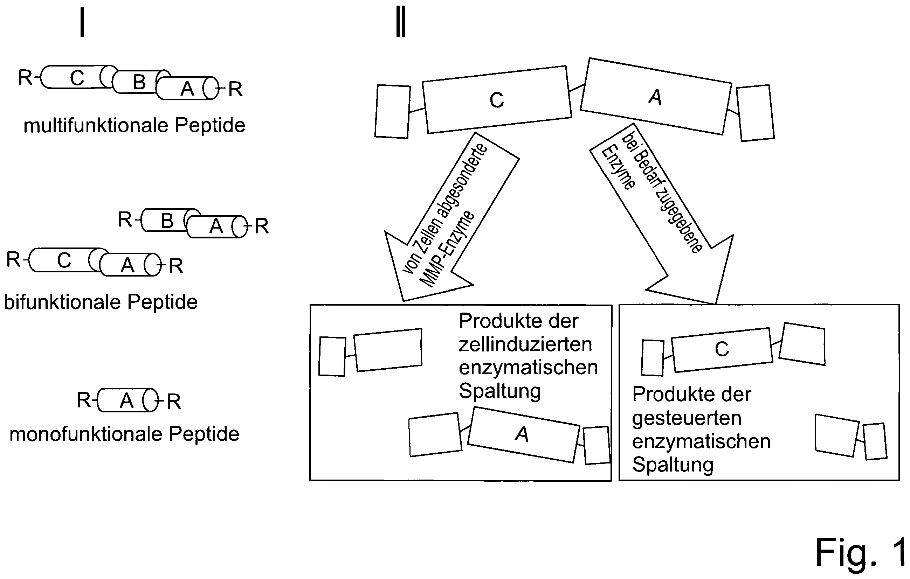

Die vorliegende Erfindung betrifft ein Verfahren zur bedarfsweisen, das heißt zeitlich gesteuerten Spaltung der kovalenten Bindung in polymerbasierten Materialien, die in Gegenwart von lebenden Zellen, Geweben oder Organen in *vitro* oder *in vivo* bioorthogonal ausgeführt wird, wie es die Fig. 1 in schematischer Form darstellt. Dieses Verfahren basiert auf dem Einbau von kurzen enzymatisch spaltbaren Peptidsequenzen als molekulare Linker, das heißt Verbindungsmoleküle, in gebräuchliche polymerbasierte Materialstrukturen, wie Hydrogele oder Kondensationspolymere.

Die Abbildung I der **Fig. 1** zeigt schematisch ein multifunktionales Peptidmolekül mit der allgemeinen Struktur R-C-B-A-R, bifunktionale Peptidmoleküle mit den allgemeinen Strukturen R-B-A-R und R-C-A-R sowie ein monofunktionales Peptidmolekül mit der allgemeinen Struktur R-A-R, wobei R jeweils einen polymeren Rest des Materials darstellt.

Dabei ist A eine enzymatisch spaltbare Peptidsequenz, die durch eine gesteuerte Enzymzugabe gespalten werden kann. Vorzugsweise sind diese Enzyme Proteasen, wie in **Tabelle 1** aufgeführt. Ebenfalls in **Tabelle 1** sind diesen Proteasen jeweils spaltbare Peptidsequenzen zugeordnet, die durch die entsprechenden Proteasen an den Spaltungsstellen (/) gespalten werden können.

Die aufgeführten Peptide sind Beispiele für Peptide, die inert sind gegenüber der allgemeinen biologischen und metabolischen Aktivität des lebenden Gewebes, aber einer selektiven enzymatischen Spaltung durch spezifische Enzyme unterzogen werden können. Die in **Tabelle 1** aufgeführten Enzyme können die Aminosäuresequenzen mit einer sehr hohen Selektivität spalten, sind jedoch ausschließlich inaktiv gegenüber dem Aufschluss sowohl zellulärer als auch extrazellulärer Matrixproteine.

**Tabelle 1 Proteasen**

| **Proteasen (Abkürzung)** | **spaltbare Peptidsequenz mit Spaltungsstelle (/)** |
|---|---|
| Tabakätzvirus Protease (TEV) | ENLYFQ/X |
| Humane Rhinovirus 3C-Protein (3C) | E(T)VLFQ/GP |
| Faktor Xₐ(Xₐ) | IEGR/IEGRX |
| Thr Thrombin-Protease | LVPRGS/FXRS |
| EntK Enterokinase | DDDDK/ |
| Caspase Caspase-3 | DXXD/ |
| PreScission™ | LEVLFQ/GP |
| Sortase A | LPET/G |
| Granzym B Serin (hohes Risiko für die unspezifische Spaltung) | DX, NX, MN, SX |

Der Buchstabe B in den Strukturen der **Fig. 1** steht für bioaktive Peptidmotive, die verschieden sind von den enzymatisch spaltbaren Peptidsequenzen A, zum Beispiel eine zelladhäsive RGD-Sequenz.

Der Buchstabe C in den Strukturen der **Fig. 1** zeigt bioaktive Peptidmotive an, die verschieden sind von den enzymatisch spaltbaren Peptidsequenzen A, zum Beispiel eine zellresponsive MMP-Sequenz, das heißt einer Peptidsequenz, die durch Matrix-Metalloprotease spaltbar ist.

Die Abbildung II der **Fig. 1** zeigt ein Modul vom Typ C als zellresponsive MMP-Sequenz und ein orthogonales enzymatisch spaltbares Modul mit einer Peptidsequenz vom Typ A, die einerseits miteinander und andererseits mit jeweils einem von zwei Materialmodulen verbunden sind. Wie die **Fig. 1** in Abbildung II zeigt, können von der Zelle ausgeschiedene MMP-Enzyme für eine Spaltung der zellresponsiven Sequenz des Moduls C sorgen. Als Produkte zeigt die schematische Ansicht der Abbildung II der **Fig. 1** ein Spaltprodukt der MMP-Sequenz C zusammen mit einem Materialmodul sowie die nicht gespaltene Peptidsequenz A, die einerseits mit dem zweiten Spaltprodukt der MMP-Sequenz und andererseits mit dem zweiten Materialbindungsmodul verbunden ist.

Eine orthogonale Spaltung der enzymatisch spaltbaren Peptidsequenz A zeigt die rechte Seite der Abbildung II in **Fig. 1****.** Eine gesteuerte Zugabe eines Enzyms, das heißt einer Zugabe des Enzyms bei Bedarf, führt hier zur orthogonalen Spaltung des enzymatisch spaltbaren Moduls A und somit zu einer Beschleunigung des Abbaus des Materials. Als Spaltprodukt entsteht zum einen ein Teil mit einem Peptidmotiv der enzymatisch gespaltenen Peptidsequenz A, das mit einem Materialbindungsmodul verbunden ist. Als zweites Spaltprodukt entsteht ein Peptidmotiv der enzymatisch gespaltenen Peptidsequenz A, das an die Sequenz C gebunden ist, die ihrerseits mit einem Materialmodul verbunden ist.

Die erfindungsgemäß verwendeten enzymatisch spaltbaren Peptide A sind inert gegenüber der allgemeinen biologischen und metabolischen Aktivität des lebenden Gewebes, können aber einer enzymatischen Spaltung durch spezifische Enzyme, wie in **Tabelle 1** gezeigt, unterzogen werden. Die enzymatische Spaltung des Materials beeinträchtigt nicht die extrazelluläre Matrix sowie Zell-Zell und extrazelluläre Matrix-Zell-Kontakte. Somit ermöglicht sie die Trennung der lebenden Probe und ihrer extrazellulären Matrix aus dem Material-Träger durch Materialabbau nach Bedarf an einer beliebigen Stelle der Entwicklung der lebenden Probe. Im Folgenden sind auch verschiedene Synthesewege für die Aufnahme einer spaltbaren Peptidsequenz in gebräuchliche weiche und kondensierte polymerbasierte Materialien beschrieben, die eine solchen Einbau in den meisten Materialien für biologische Anwendungen ermöglicht.

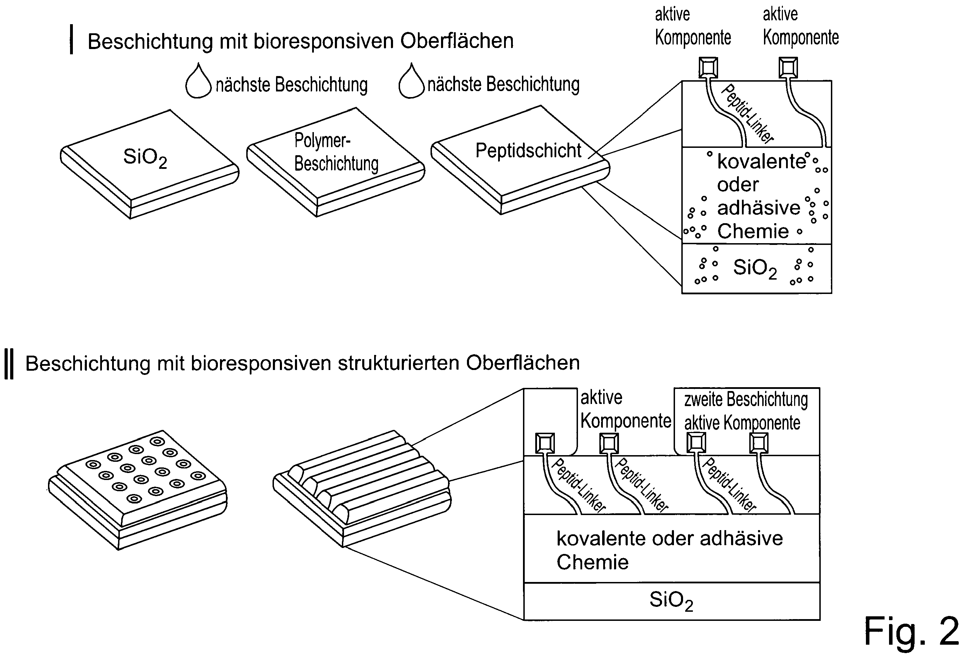

Wie die **Fig. 2** zeigt, kann ein Linker mit einer enzymatisch spaltbaren Peptidsequenz als ein Teil einer Beschichtung aufgenommen werden, wobei die spaltbare Peptidsequenz das Ablösen eines jeden Objektes, das an die Beschichtung über den Linker angebracht ist, erlaubt.

Die **Fig. 2** zeigt in Abbildung I schematisch die Beschichtung eines SiO₂-Grundsubstrats auf bekanntem Wege zunächst mit einer oder mehreren funktionalisierten Polymerbeschichtungen mit kovalenter oder adhäsiver Anbindung (kovalente oder adhäsive Chemie), auf die dann eine Beschichtung mit Peptiden erfolgt. Die Peptide mit den spaltbaren Peptidsequenzen verbinden als molekulare Linker die funktionellen Gruppen der Polymerschicht mit einer aktiven Verbindung.

Wie die untere Abbildung II in **Fig. 2** zeigt, kann durch eine zweite teilweise Beschichtung eine strukturierte bioresponsive Oberfläche erzeugt werden, indem in einer Beschichtung nur ein Teil der Peptidbeschichtung mit den aktiven Verbindungen an der Oberfläche unbeschichtet ist.

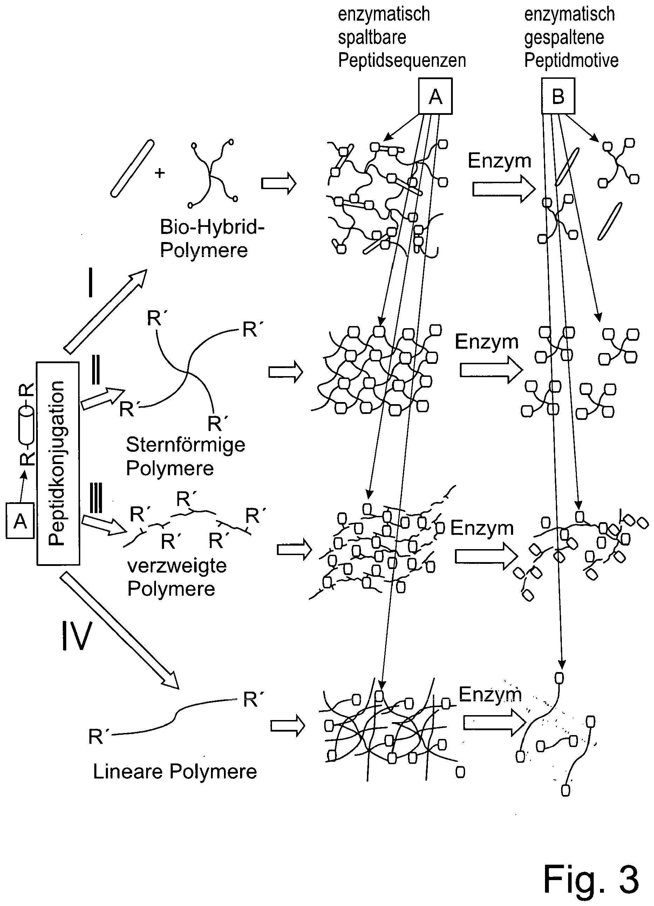

Die **Fig. 3** zeigt anhand von Beispielen I, II, III und IV schematisch den Einbau von enzymatisch spaltbaren Peptid-Linkern in ein Hydrogel oder ein kondensiertes polymeres Material. Wie die Beispiele in **Fig. 3** zeigen, ist der enzymatisch spaltbare Linker Teil des Hydrogels beziehungsweise des kondensierten polymeren Materials, wobei der spaltbare Linker bei Bedarf den gesteuerten Abbau des Hydrogels beziehungsweise der Struktur des kondensierten polymeren Materials erlauben. In Folge des Abbaus werden dabei Objekte, die an das Material gebunden oder in das Material eingeschlossen sind, freigesetzt.

Das Beispiel I in **Fig. 3** zeigt schematisch die Bildung eines Hydrogels/Kondensationspolymers aus Biohybrid-Polymeren. Biohybrid-Polymere können durch die Kopplung von nichtbiologischen Polymeren, wie zum Beispiel Polyethylenglykol (PEG) oder Polyacrylamid, mit biologischen Komponenten wie Sacchariden, Proteinmodulen und/oder DNA-Elementen hergestellt werden. Das Beispiel I zeigt dabei ein sternförmiges Polymer, an dessen Enden sich jeweils eine Peptidsequenz befindet. Die kovalent gebundenen, enzymatisch abbaubaren Peptidsequenzen die Biohybrid-Polymeren miteinander, indem jeweils biologische Komponente an mehrere Peptidsequenzen angebracht ist, die sich an den Enden der sternförmigen Polymermoleküle befinden. Die spaltbaren Peptidsequenzen sind somit Teil des Hydrogels. Die biologischen Komponenten sind somit an das Material angebracht beziehungsweise in das Material eingeschlossen. Durch die Zugabe eines Enzyms und die dadurch gesteuerte Spaltung der Peptidsequenz vom biologischen Material erfolgt ein Abbau des Hydrogelmaterials, wodurch die biologische Komponente freigesetzt wird und die Peptidsequenzen an den nichtbiologischen Molekülen verbleiben.

Das Beispiel II der **Fig. 3** stellt die Erzeugung eines polymerbasiertes Material in eines Hydrogel oder Kondensationspolymers aus sternförmigen Polymeren, zum Beispiel Stern-PEG, schematisch dar, bei dem die enzymatisch spaltbaren Peptidsequenzen die Enden der Vielzahl an sternförmigen Polymermolekülen zu einer Netzwerkstruktur, die Knoten und Kanten bildet, verbinden. Durch Enzymzugabe und die dadurch veranlasste Spaltung der Peptidsequenzen kann ein gesteuerter Abbau der Netzwerkstruktur in einzelne sternförmige Polymere erfolgen, an deren Enden sich jeweils die Reste der gespaltenen Peptidsequenz in Form von Peptidmotiven befinden.

Im Beispiel III der **Fig. 3** sind die enzymatisch spaltbaren Peptidsequenzen Teil eines Hydrogels/Kondensationspolymers mit verzweigten Polymeren mit kurzen Seitenketten, wobei die verzweigten Moleküle an den Enden ihrer Seitenketten über die spaltbaren Peptidsequenzen miteinander verbunden sind. Durch Enzymzugabe und die dadurch veranlasste Spaltung der Peptidsequenzen kann ein gesteuerter Abbau der Netzwerkstruktur in einzelne verzweigte Polymere mit Peptidmotiven der gespaltenen Peptidsequenzen an den Enden der Seitenketten erfolgen.

In Beispiel IV gemäß **Fig. 3** sind die enzymatisch spaltbaren Peptidsequenzen Teil eines Hydrogels/Kondensationspolymers mit linearen Polymeren, wobei die linearen Moleküle an den Enden ihrer Seitenketten über die spaltbaren Peptidsequenzen miteinander verbunden sind und ein Knäuel gebildet wird. Durch Enzymzugabe und die dadurch veranlasste Spaltung der Peptidsequenzen kann ein gesteuerter Abbau der Knäuelstruktur in einzelne lineare Polymere erfolgen, an deren Enden sich Peptidmotive der gespaltenen Peptidsequenzen befinden.

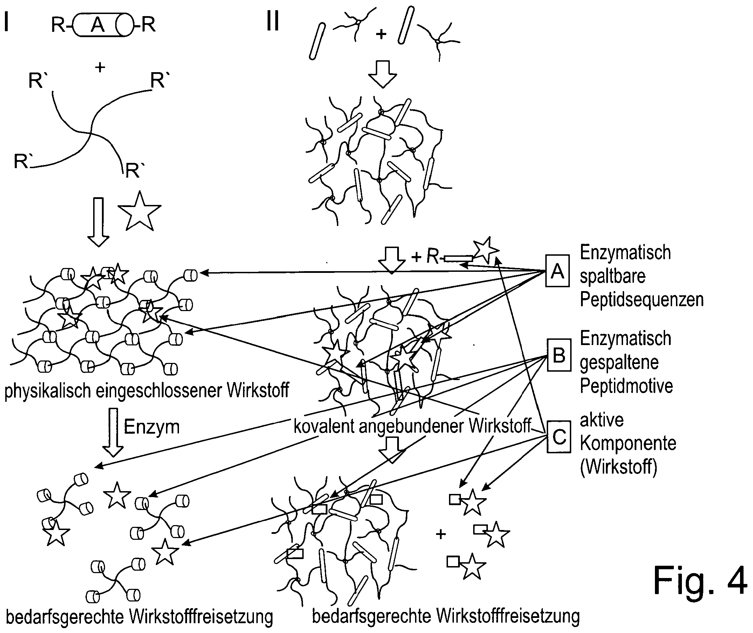

Die **Fig. 4** zeigt schematisch die Anwendung der Erfindung für die Freisetzung von Wirkstoffen, die an das Material durch einen molekularen Linker mit einer spaltbaren Peptidsequenz gebunden sind. Dies erlaubt eine bedarfsgerechte, zeitlich gesteuerte Abgabe des Wirkstoffes, wobei der Wirkstoff an der Oberfläche des Materials oder in das Materialgerüst als dreidimensionale Anwendung angebracht ist.

Die Abbildung I der **Fig. 4** zeigt schematisch die Erzeugung eines polymerbasierten Materials in Form eines Hydrogels oder Kondensationspolymers, bei dem die Moleküle eines sternförmigen Polymers, zum Beispiel Stern-PEG, durch Linkermoleküle, die enzymatisch spaltbare Peptidsequenzen enthalten, an den Enden der Polymermoleküle zu einer dreidimensionalen Netzwerkstruktur verbunden sind. Durch Zugabe eines Wirkstoffs bei der Erzeugung der Netzwerkstruktur werden die Wirkstoffmoleküle physikalisch in der Netzwerkstruktur eingeschlossen. Durch Enzymzugabe und die dadurch veranlasste Spaltung der Peptidsequenzen kann dann ein gesteuerter Abbau der Netzwerkstruktur in einzelne sternförmige Polymere erfolgen, an deren Enden sich jeweils die Reste der gespaltenen Peptidsequenz in Form von Peptidmotiven befinden. Durch den Abbau der Netzstruktur werden auch die zuvor in die Netzstruktur physikalisch eingeschlossenen Wirkstoffe freigesetzt.

Die Abbildung II zeigt in **Fig. 4** schematisch die Bildung eines dreidimensionalen vernetzten Materials aus Biohybrid-Polymeren, vorzugsweise einem Hydrogel oder einem Kondensationspolymer, an dessen Oberfläche Moleküle, die jeweils eine enzymatisch spaltbare Peptidsequenz mit einem kovalent angebundenen Wirkstoff enthalten, angebracht werden. Durch die Zugabe des Enzyms erfolgt die Spaltung der kovalenten Bindung, was zur Freisetzung der Wirkstoffmoleküle mit kovalent gebundenen Peptidmotiven der gespaltenen Peptidsequenzen führt. Auf diese Weise ist eine mittels Enzymzugabe gesteuerte Freisetzung des Wirkstoffs möglich, wobei das dreidimensionale Netzwerk (Hydrogel oder Kondensationspolymer) erhalten bleibt.

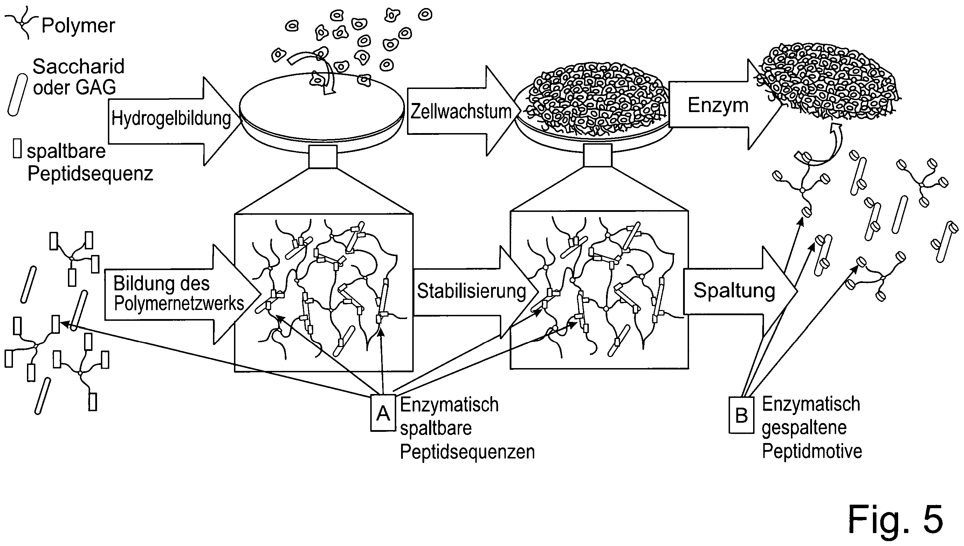

Das Verfahren eines bioorthogonalen Materialabbaus kann auch für die Herstellung von Zell- und Gewebeeinzelschichten und Transplantaten oder in dreidimensionalen Anwendungen genutzt werden **(****Fig. 5****).**

Die **Fig. 5** zeigt eine schematische Darstellung der Anwendung des bioorthogonalen Materialabbaus für die Herstellung von Zell-, Gewebeeinzelschichten oder Transplantaten, bei dem das anzubauende Material ein Hydrogel ist. Dieses Hydrogel umfasst ein Polymernetzwerk, welches, wie in der **Fig. 5** gezeigt, durch Mischen einer sternförmigen polymeren Komponente, einer zweiten Komponente in Form eines Saccharides oder eines Glykosaminglykans (GAGs) sowie einer spaltbaren kurzen Peptidsequenz erzeugt wird. Zusammen bilden diese drei Komponenten ein Polymernetzwerk, bei dem sich an den Enden des sternförmigen Polymers jeweils eine Peptidsequenz befindet. An jedes Saccharid- oder GAG-Molekül sind jeweils mehrere Peptidsequenzen, die nicht mit demselben Polymermolekül verbunden sind, gebunden. Auf diese Weise wird das Polymernetzwerk für das Hydrogel gebildet, dessen Teil die spaltbaren Peptidsequenzen sind. Die spaltbaren Peptidsequenzen sind als Teil der dreidimensionalen Struktur des polymerbasierten Materials für das Bestehen der Struktur essentiell, sodass im Falle der Spaltung beziehungsweise des Abbaus der Bindung der Peptidsequenzen ein teilweiser bis vollständiger bioorthogonaler Abbau der Struktur erfolgt. Nach der Hydrogelbildung werden, wie im oberen Teil der **Fig. 5** gezeigt, Zellen auf das Hydrogel aufgebracht, wodurch es zum Zellwachstum kommt. Nach der Bildung der Zell- oder Gewebeschicht beziehungsweise der Transplantatherstellung kann die Haftverbindung zwischen dem polymerbasierten Material und der Zellschicht/dem Gewebe/dem Transplantat/Implantat abgebaut werden, indem eine gesteuerte Enzymzugabe erfolgt. Das Enzym sorgt für die Spaltung/den Abbau einer kovalenten Bindung der Peptidsequenzen, was einen bioorthogonalen Abbau der dreidimensionalen Materialstruktur zur Folge hat. Das Polymernetzwerk zerfällt dabei in in sternförmige Polymermoleküle mit endständigen Peptidmotiven der zuvor gespaltenen Peptidsequenz sowie Saccharid oder GAG-Moleküle, ebenfalls mit angebundenen Peptidmotiven der gespaltenen Peptidsequenz, womit das sternförmige Polymer sowie das Saccharid oder GAG von der Zell- oder Gewebeschicht beziehungsweise dem Transplantat/Implantat schonend und selektiv abgelöst werden.

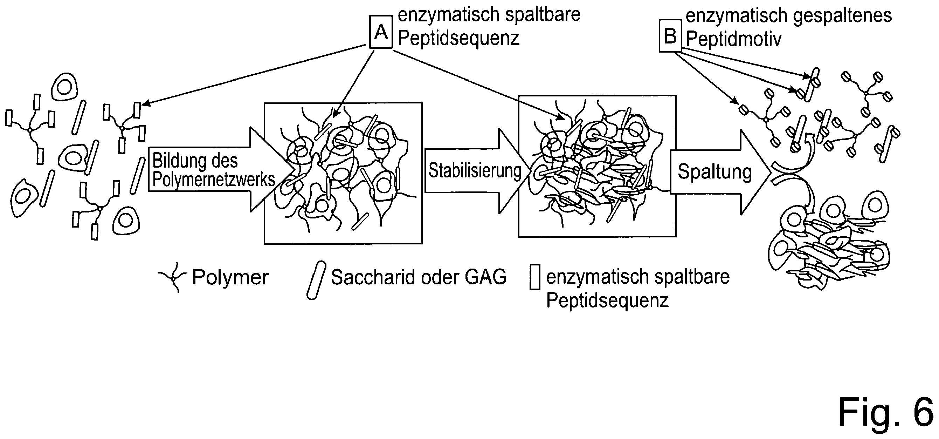

Die **Fig. 6** verdeutlicht in Form einer schematischen Darstellung die Anwendung des bioorthogonalen Materialabbaus für die Zell- und Gewebeherstellung in dreidimensionalen Anwendungen. Dabei wird ein Polymernetzwerk durch Mischen einer sternförmigen polymere Komponente, an deren vier Enden sich jeweils eine kurze spaltbare Peptidsequenz befindet, sowie eines Saccharides oder eines GAGs erzeugt. An jedes Saccharid- oder GAG-Molekül sind im Netzwerk jeweils mehrere Peptidsequenzen, die nicht mit demselben Polymermolekül verbunden sind, gebunden (Bildung eines Netzwerkes). Durch Zugabe von Zellen bei der Erzeugung der Netzwerkstruktur werden die Zellen physikalisch in die Netzwerkstruktur eingebunden. Die dreidimensionale Netzwerkstruktur bietet ein stabiles Gerüst für die Zellen, welches der dreidimensionalen extrazellulären Matrix ähnlich ist. Nach erfolgreicher Herstellung des Gewebes kann dieses durch die gesteuerte enzymatische Spaltung der Peptidsequenzen schonend und selektiv vom dreidimensionalen Trägermaterial abgetrennt werden, wobei das dreidimensionale Netzwerk in sternförmige Polymermoleküle mit endständigen Peptidmotiven der zuvor gespaltenen Peptidsequenz sowie Saccharid oder GAG-Moleküle, ebenfalls mit angebundenen Peptidmotiven der gespaltenen Peptidsequenz, zerfällt.

Diese Erfindung stellt ein neues Werkzeug für Forscher oder medizinisches Personal bei der Entnahme, Lagerung sowie Kultur/Züchtung von lebenden Zellen, Geweben, Organen bereit, die bei anderen Methoden nicht zur Verfügung stehen.

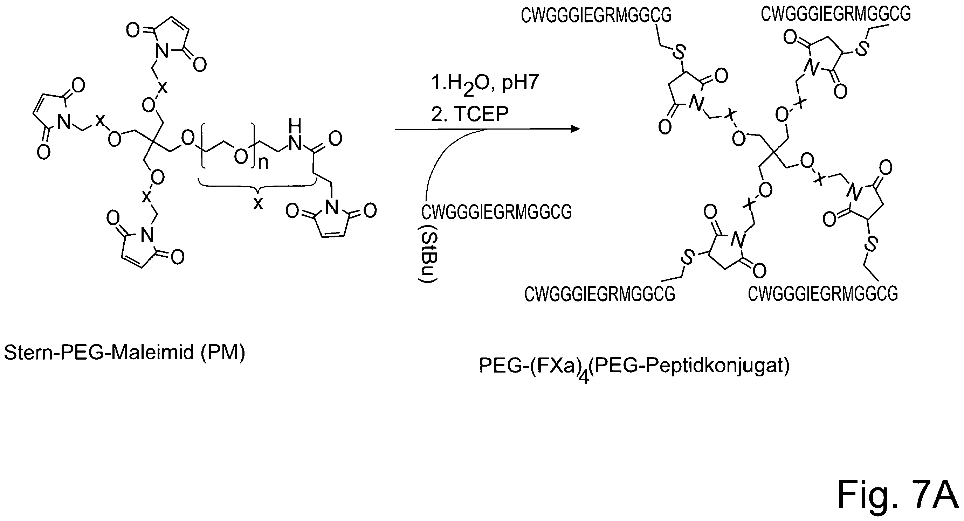

Im Folgenden werden weitere Ausführungsbeispiele detailliert beschrieben:
Es erfolgte eine Synthese von **PEG-(FXa)₄** -Konjugaten, wie in **Fig. 7A** schematisch dargestellt. Die Synthese der **PEG-(FXa)₄** -Konjugate wurde ausgeführt, wie es auch früher in der Druckschrift Tsurkan M. V. et al. Defined polymer-peptide conjugates to form cell-instructive starPEG-heparin matrices in situ. Adv. Mater. 25, 2606-2610 (2013**),** beschrieben wurde. Unter FXa-Peptid ist ein Peptid zu verstehen, das eine durch Faktor Xₐ-Protease spaltbare Stelle aufweist.

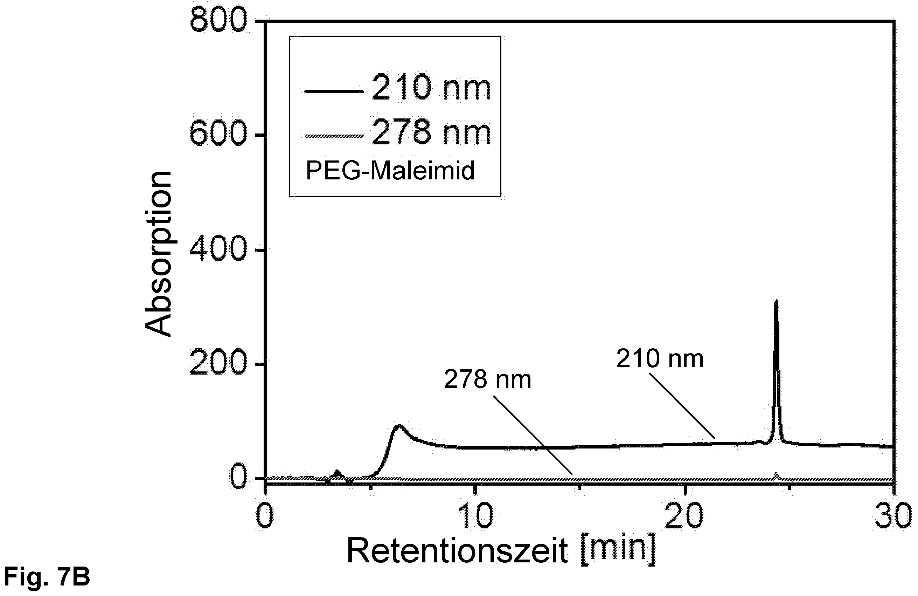

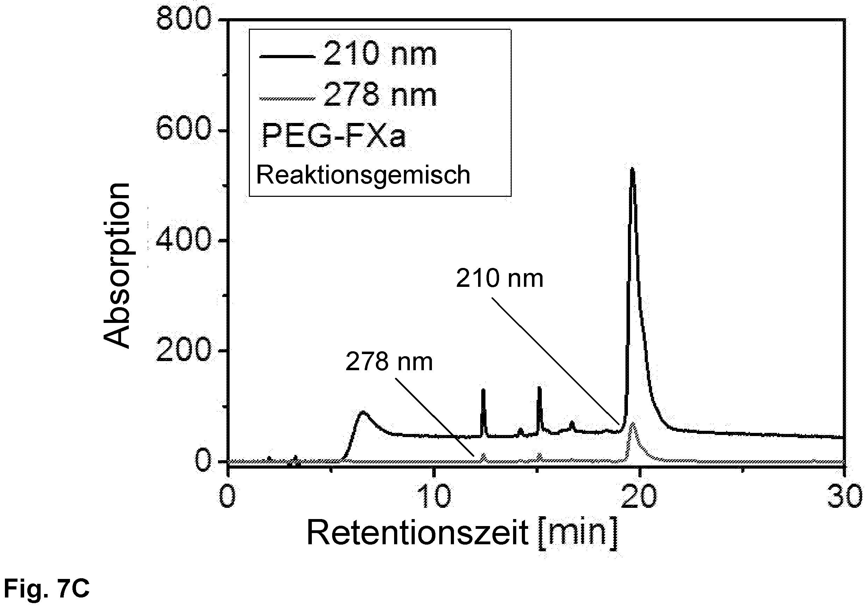

Kurzzeitig wurden 427 mg von PEG-Maleimid in 10 mL 50%-iger (v/v) Acetonitril/ Wasser gelöst und mit 300 mg des FXa-Peptids (30% Überschuss), das in 15 mL 50%-iger (v/v) Acetonitril/ Wasser gelöst war, gemischt. Der pH-Wert der Reaktionsmischung wurde durch 1 M NaOH auf pH 7,5 - 8 eingestellt. Die Reaktion wurde für 5 Stunden unter Stickstoff-Atmosphäre durchgeführt, und die Beendigung der Reaktion wurde über eine analytische HPLC verfolgt. Das Reaktionsgemisch wurde durch präparative HPLC gereinigt. Das Produkt wurde aus der HPLC gesammelt und für mehr als 24 Stunden gefriergetrocknet. Das gebildete weiße Pulver wurde dann bei -20 °C gelagert. Das SyntheseSchema und die HPLC-Überwachung der Reaktion sind in den Figuren **Fig. 7A****,** **Fig. 7B** und **Fig 7C** angegeben.

### Hydrogelbildung:

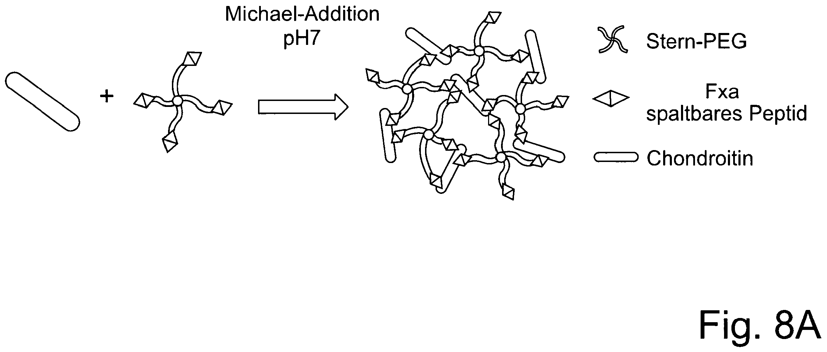

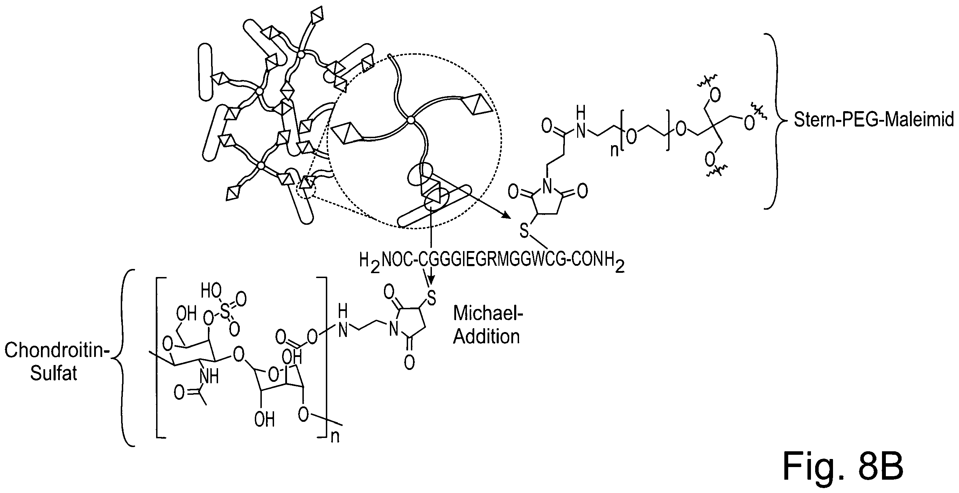

Die schematische Darstellung der Hydrogelbildung und die mechanischen Eigenschaften als Funktion des Hydrogel-Vernetzungsgrades sind in den Figuren **Fig. 8A** und **Fig. 8** **B** gezeigt. Das Hydrogelnetzwerk wird durch eine Michael-Additionsreaktion unter Ausbildung von Peptidvernetzungspunkten (Knoten) gebildet. Der Gesamtfeststoffgehalt aller Gelgemische wurde unabhängig von dem Vernetzungsgrad (was einem Molverhältnis PEG / CSMal₆ = γ entspricht) immer bei 5% (50 mg/ml) gehalten. Eine einfache Einstellung des Volumenverhältnisses der PEG-(FXa)₄ und CSMal₆, bei Beibehaltung eines konstanten Gesamtvolumens, ermöglicht die Einstellung der Schwellung und Steifigkeit der gebildeten Hydrogele. CS ist dabei die Kurzbezeichnung für Chondroitinsulfat, Mal die Kurzbezeichnung für Maleimid.

### Bildung von HCEC-Schichten während des Hydrogelabbaus:

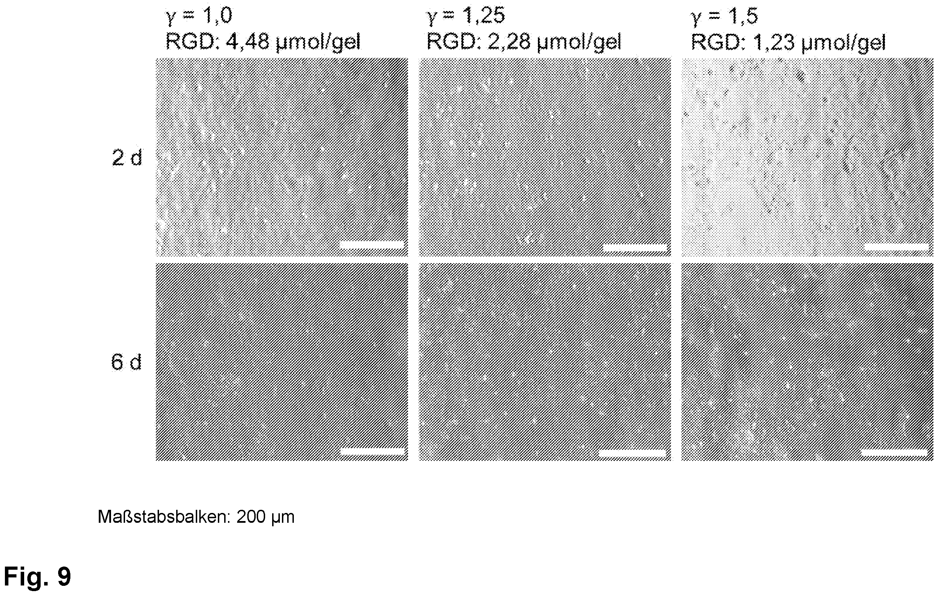

Die immortalisierten menschlichen Hornhautendothelzellen der (HCEC)-Linie HCEC-B4G12, wie in der Druckschrift Valtink M. et al. Two clonal cell lines of immortalized human corneal endothelial cells show either differentiated or precursor cell characteristics. Cells. Tissues. Organs 187, 286-94 (2008**)** beschrieben, wurde in humanem Endothel-SFM (SFM = Serum-freies Hornhaut-Organkulturmedium), ergänzt mit 10 ng/ml menschlichem rekombinanten basischen Fibroblasten-Wachstumsfaktor (bFGF), kultiviert. HCEC wurden mit einer Dichte von 1 x 10⁵ Zellen pro cm² auf die FXa-abbaubaren Hydrogele ausgesät. Die Zellen wurden bei 37 ° C in einer feuchten Atmosphäre gehalten, die 5% CO₂ enthielt. Das Medium wurde dreimal pro Woche gewechselt. Die Hydrogelbildung und die mechanischen Eigenschaften in Abhängigkeit des Hydrogel-Vernetzungsgrades sind in der **Fig. 9** gezeigt. Die **Fig. 9** zeigt mikroskopische Aufnahmen der Langzeit-HCEC-Kultur auf FXa-abbaubaren Hydrogelen. FXa-spaltbare Hydrogele des Vernetzungsgrades y = 1,25 wurden als geeignet für das HCEC-Kultivierungsverfahren ermittelt, da nach 48 Stunden (2d) eine nahezu zusammengewachsene Zellschicht gebildet wurde.

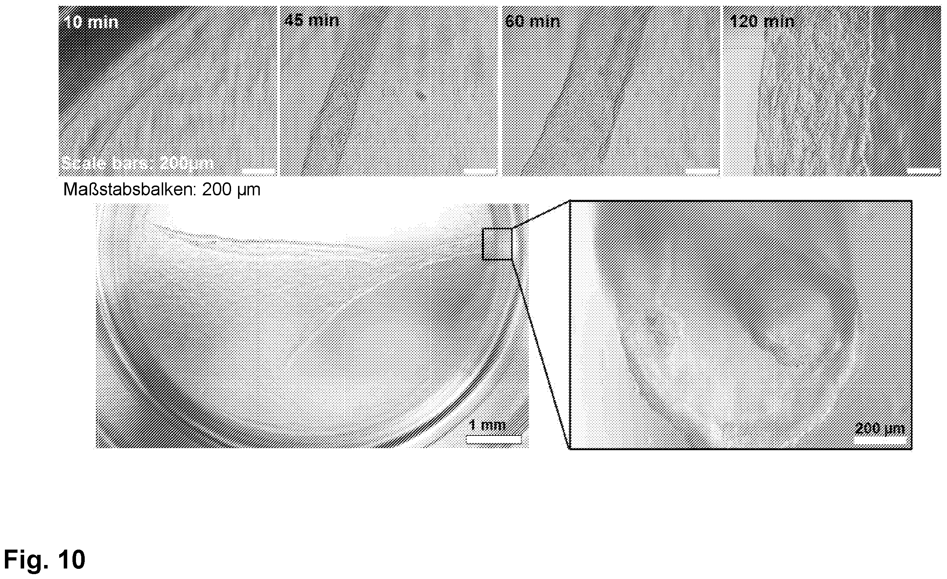

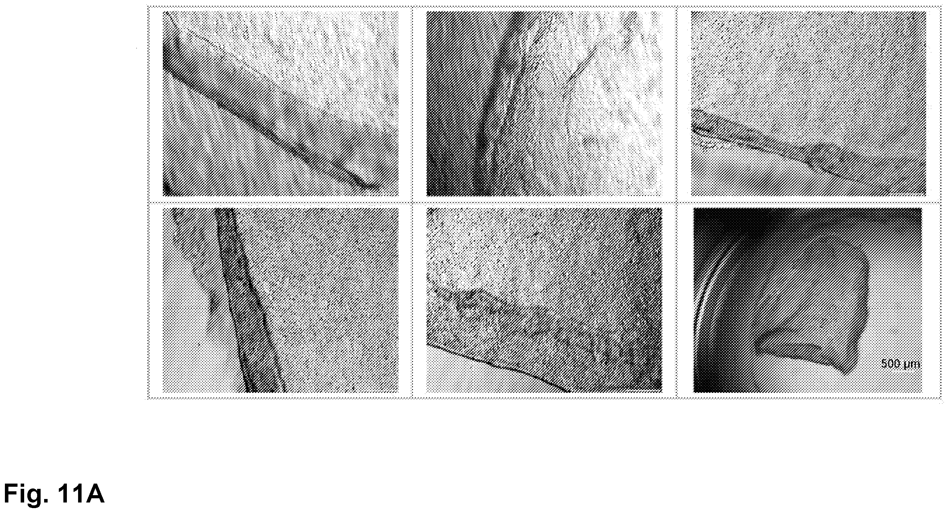

Nach sieben Tagen Kultivierung auf den FXa-abbaubaren Hydrogelen wurden die Proben in humanem Endothel-SFM w/ 900 nM Faktor Xa Endoprotease für 45 min bei 37 ° C inkubiert. Nach dieser Zeit waren die zellulären Monolagen vollständig freigesetzt und konnten sorgfältig mit einer 20-Gauge-Kanüle (HSW Fine Ject, Tuttlingen, Deutschland) behandelt werden. Die lichtmikroskopischen Abbildungen der HCEC-Schichtbildung während des Hydrogelabbaus sind in den ergänzenden Figuren **Fig. 10** und **Fig. 11A** dargestellt.

Die **Fig. 10** zeigt mittels lichtmikroskopischer Aufnahmen die Bildung von HCEC-Schichten während des Hydrogelabbaus in humanem Endothel-SFM w/ 450 nM Faktor-Xa Endoprotease bei 37°C. Eine vollständige Degradierung konnte nach 120 min beobachtet werden.

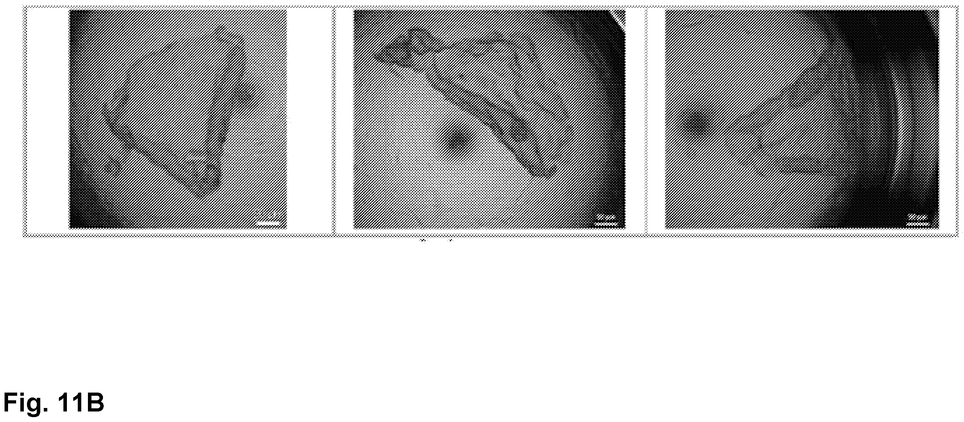

Die **Fig. 11** **A** zeigt mittels lichtmikroskopischer Aufnahmen die Bildung von HCEC-Schichten während des Hydrogelabbaus in humanem Endothel-SFM w/ 900 nM Faktor-Xa Endoprotease bei 37°C. Eine vollständige Degradierung fand nach 45 min statt.

Die **Fig. 11B** zeigt verschiedene lichtmikroskopische Aufnahmen von verschiedenen Beispielen der gebildeten HCEC-Schicht.

## Patentansprüche

1. Polymerbasiertes Material mit kovalent gebundenen, enzymatisch abbaubaren Peptidsequenzen, die zumindest eine durch Proteasen enzymatisch spaltbare Stelle aufweisen, die ausgewählt ist aus einer Gruppe von spaltbaren Stellen, bestehend aus einer durch Tabakätzvirus-Protease spaltbaren Stelle, einer durch humane Rhinovirus-3C-Protease spaltbaren Stelle, einer durch Faktor Xₐ-Protease spaltbaren Stelle, einer durch Thrombin-Protease spaltbaren Stelle, einer durch Enterokinase spaltbaren Stelle, einer durch Sortase-A-Protease spaltbaren Stelle, einer durch Caspase-3-Protease spaltbaren Stelle, einer durch Granzym-B-Serin-Protease spaltbaren Stelle, einer durch ein Fusionsprotein aus Glutathion-S-Transferase (GST) und Humaner-Rhinovirus(HRV)-Typ-14-3C-Protease spaltbaren Stelle, wobei die Peptidsequenzen jeweils aus zwei bis fünfzehn Aminosäuren bestehen und derart in das polymerbasierte Material eingebaut oder an das polymerbasierte Material konjugiert sind, dass sie entweder Verbindungsmoleküle (Linker) in einer zwei- oder dreidimensionalen Struktur des polymerbasierten Materials sind, so dass mittels eines durch Enzym-Zugabe gesteuerten Abbaus einer kovalenten Bindung der Peptidsequenz entweder ein bioorthogonaler Abbau der dreidimensionalen Struktur, oder im Falle einer Konjugation der Peptidsequenz an das polymerbasierte Material die Freisetzung zumindest eines Teils des Moleküls erfolgt.

2. Polymerbasiertes Material nach Anspruch 1, **dadurch gekennzeichnet, dass** das polymerbasierte Material ein Hydrogel oder ein kondensiertes Polymer ist.

3. Polymerbasiertes Material nach Anspruch 1 oder 2, **dadurch gekennzeichnet, dass** das polymerbasierte Material mindestens eine bioaktive Komponente umfasst, die durch einen mittels Enzym-Zugabe gesteuerten Abbau der kovalenten Bindung der Peptidsequenz freisetzbar ist und die einer Gruppe von Komponenten angehört, bestehend aus Wirkstoffen, Nukleinsäuren (DNA, RNA, Aptamere), Proteinen, Peptid-Konjugaten, sulfatierten und nicht sulfatierten Polysacchariden und deren Konjugaten.

4. Polymerbasiertes Material nach Anspruch 3, **dadurch gekennzeichnet, dass** die Moleküle der mindestens einen freisetzbaren Komponente durch Linker, die die enzymatisch abbaubare Peptidsequenz jeweils enthalten, an das polymerbasierte Material konjugiert sind.

5. Polymerbasiertes Material nach Anspruch 3, **dadurch gekennzeichnet, dass** die Moleküle der mindestens einen freisetzbaren Komponente in ein dreidimensionales Polymernetzwerk physikalisch eingeschlossen und/oder daran physikalisch gebunden sind.

6. Polymerbasiertes Material nach einem der Ansprüche 1 bis 5, **dadurch gekennzeichnet, dass** die enzymatisch abbaubaren Peptidsequenzen durch die biologische und metabolische Aktivität von Zellen und Geweben nicht abbaubar sind.

7. Polymerbasiertes Material nach Anspruch 6, **dadurch gekennzeichnet, dass** die spaltbare Peptidsequenz, die eine Protease-spaltbare Stelle aufweist, ausgewählt wird aus einer Gruppe, bestehend aus ENLYFQ/X, ETVLFQ/GP, EVLFQ/GP, IEGR/IEGRX, CWGGGIEGR/MGGCG, CGGGIEGR/MGGWCG, DDDDK/, LVPRGS/FXRS, DXXD/, LEVLFQ/GP, LPET/G, DX, NX, MN und SX, wobei der Schrägstrich (/) jeweils die spaltbare Stelle anzeigt, die Buchstaben den Einbuchstabencode der proteinogenen Aminosäuren und X eine beliebige natürlich vorkommende Aminosäure darstellen.

8. Polymerbasiertes Material nach Anspruch 6 oder 7, ferner umfassend Peptidsequenzen, die durch die biologische und metabolische Aktivität von Zellen und Geweben abbaubar sind, und derart in das polymerbasierte Material eingebaut oder an das polymerbasierte Material konjugiert sind, dass sie entweder Verbindungsmoleküle (Linker) in einer zwei- oder dreidimensionalen Struktur des polymerbasierten Materials sind, so dass durch die biologische und metabolische Aktivität von Zellen und Geweben ein bioorthogonaler Abbau der dreidimensionalen Struktur, oder im Falle einer Konjugation der Peptidsequenz an das polymerbasierte Material die Freisetzung zumindest eines Teils des Moleküls erfolgt.

9. Polymerbasiertes Material nach einem der Ansprüche 3 bis 8 zur Verwendung für eine gesteuerte *in-vivo*-Freisetzung der freisetzbaren Komponente, die durch einen mittels Zugabe der Protease gesteuerten Abbau der kovalenten Bindung der Peptidsequenz erfolgt.

10. Polymerbasiertes Material zur Verwendung nach Anspruch 9 für eine *in-vivo*-Behandlung von Zellen, Geweben und Organen, bei der bei Bedarf ein Wirkstoff und/oder eine andere der genannten freisetzbaren bioaktiven Komponenten durch Zugabe der Protease gesteuert freigesetzt wird/werden.

11. Verwendung eines polymerbasierten Materials nach einem der Ansprüche 1 bis 8 für eine in-vitro-Zellkultur oder *in-vitro*-Herstellung von Geweben oder Organen.

12. Verwendung eines polymerbasierten Materials nach einem der Ansprüche 1 bis 8 für die *in-vitro*-Stabilisierung von gespendeten Zellen, Geweben und Organen.

13. Verwendung eines polymerbasierten Materials nach einem der Ansprüche 3 bis 8 für ein *in-vitro*-Verfahren, bei dem bei Bedarf ein mittels Zugabe der Protease gesteuerter Abbau der kovalenten Bindung der Peptidsequenz und damit eine Freisetzung der freisetzbaren Komponente erfolgt.

14. Verwendung nach Anspruch 13 bei der *in-vitro*-Behandlung von lebenden Zellen, Geweben oder Organen, wobei bei Bedarf ein Wirkstoff und/oder eine andere der genannten freisetzbaren bioaktiven Komponenten durch Zugabe der Protease gesteuert freigesetzt wird/werden.

## Claims

1. Polymer-based material having covalently bonded, enzymatically degradable peptide sequences which have at least one site which is enzymatically cleavable by proteases and which is selected from a group of cleavable sites consisting of a site cleavable by tobacco etch virus protease, a site cleavable by human rhinovirus 3C protease, a site cleavable by factor Xₐ protease, a site cleavable by thrombin protease, a site cleavable by enterokinase, a site cleavable by sortase A protease, a site cleavable by caspase 3 protease, a site cleavable by granzyme B serine protease, a site cleavable by a fusion protein composed of glutathione S-transferase (GST) and human rhinovirus (HRV) type 14 3C protease, wherein the peptide sequences each consist of two to fifteen amino acids and are incorporated in the polymer-based material or conjugated to the polymer-based material such that they are connecting molecules (linkers) in a two-dimensional or three-dimensional structure of the polymer-based material, with the result that an enzyme addition-controlled degradation of a covalent bond of the peptide sequence causes either a bioorthogonal degradation of the three-dimensional structure or, in the case of a conjugation of the peptide sequence to the polymer-based material, the release of at least a portion of the molecule.

2. Polymer-based material according to Claim 1, **characterized in that** the polymer-based material is a hydrogel or a condensed polymer.

3. Polymer-based material according to Claim 1 or 2, **characterized in that** the polymer-based material comprises at least one bioactive component which is releasable by an enzyme addition-controlled degradation of the covalent bond of the peptide sequence and which belongs to a group of components consisting of active ingredients, nucleic acids (DNA, RNA, aptamers), proteins, peptide conjugates, sulfated and non-sulfated polysaccharides and the conjugates thereof.

4. Polymer-based material according to Claim 3, **characterized in that** the molecules of the at least one releasable component are conjugated to the polymer-based material by linkers which each contain the enzymatically degradable peptide sequence.

5. Polymer-based material according to Claim 3, **characterized in that** the molecules of the at least one releasable component are physically embedded in a three-dimensional polymer network and/or physically bound thereto.

6. Polymer-based material according to any of Claims 1 to 5, **characterized in that** the enzymatically degradable peptide sequences are not degradable by the biological and metabolic activity of cells and tissues.

7. Polymer-based material according to Claim 6, **characterized in that** the cleavable peptide sequence having a protease-cleavable site is selected from a group consisting of ENLYFQ/X, ETVLFQ/GP, EVLFQ/GP, IEGR/IEGRX, CWGGGIEGR/MGGCG, CGGGIEGR/MGGWCG, DDDDK/, LVPRGS/FXRS, DXXD/, LEVLFQ/GP, LPET/G, DX, NX, MN and SX, where the slash (/) indicates the cleavable site in each case, the letters are the one-letter code of the proteinogenic amino acids and X is an arbitrary naturally occurring amino acid.

8. Polymer-based material according to Claim 6 or 7, further comprising peptide sequences which are degradable by the biological and metabolic activity of cells and tissues and are incorporated in the polymer-based material or conjugated to the polymer-based material such that they are connecting molecules (linkers) in a two-dimensional or three-dimensional structure of the polymer-based material, with the result that the biological and metabolic activity of cells and tissues causes a bioorthogonal degradation of the three-dimensional structure or, in the case of a conjugation of the peptide sequence to the polymer-based material, the release of at least a portion of the molecule.

9. Polymer-based material according to any of Claims 3 to 8 for use for a controlled *in vivo* release of the releasable component, which is effected by a protease addition-controlled degradation of the covalent bond of the peptide sequence.

10. Polymer-based material for use according to Claim 9 for an *in vivo* treatment of cells, tissues and organs, in which an active ingredient and/or another of the stated releasable bioactive components is/are released as required in a controlled manner by protease addition.

11. Use of a polymer-based material according to any of Claims 1 to 8 for an *in vitro* cell culture or *in vitro* production of tissues or organs.

12. Use of a polymer-based material according to any of Claims 1 to 8 for the *in vitro* stabilization of donor cells, tissues and organs.

13. Use of a polymer-based material according to any of Claims 3 to 8 for an *in vitro* method in which a protease addition-controlled degradation of the covalent bond of the peptide sequence and hence a release of the releasable component is effected as required.

14. Use according to Claim 13 in the *in vitro* treatment of living cells, tissues or organs, wherein an active ingredient and/or another of the stated releasable bioactive components is/are released as required in a controlled manner by protease addition.

## Revendications

1. Matériau à base de polymère comportant des séquences peptidiques dégradables par voie enzymatique, liées de manière covalente, qui comprennent au moins un site clivable par voie enzymatique par des protéases, qui est choisi dans un groupe de sites clivables constitué par un site clivable par la protéase du virus de la gravure du tabac, un site clivable par la protéase 3C du rhinovirus humain, un site clivable par la protéase facteur Xₐ, un site clivable par la protéase thrombine, un site clivable par une entérokinase, un site clivable par une protéase sortase A, un site clivable par une protéase caspase 3, un site clivable par une protéase granzyme-B-sérine, un site clivable par une protéine de fusion de glutathione-S-transférase (GST) et une protéase de rhinovirus humain (RVH) de type 14-3C, les séquences peptidiques étant chacune constituées par deux à quinze acides aminés et étant incorporées dans le matériau à base de polymère ou conjuguées au matériau à base de polymère, de telle sorte qu'elles soient des molécules de liaison (lieurs) dans une structure bi- ou tridimensionnelle du matériau à base de polymère, de telle sorte qu'une dégradation d'une liaison covalente de la séquence peptidique, dirigée par ajout d'enzyme, permette soit une dégradation bio-orthogonale de la structure tridimensionnelle, soit en cas de conjugaison de la séquence peptidique au matériau à base de polymère la libération d'au moins une partie de la molécule.

2. Matériau à base de polymère selon la revendication 1, **caractérisé en ce que** le matériau à base de polymère est un hydrogel ou un polymère condensé.

3. Matériau à base de polymère selon la revendication 1 ou 2, **caractérisé en ce que** le matériau à base de polymère comprend au moins un composant bioactif, qui peut être libéré par une dégradation de la liaison covalente de la séquence peptidique, dirigée par ajout d'enzyme, et qui appartient à un groupe de composants constitué par les agents actifs, les acides nucléiques (ADN, ARN, aptamères), les protéines, les conjugués de peptides, les polysaccharides sulfatés et non sulfatés et leurs conjugués.

4. Matériau à base de polymère selon la revendication 3, **caractérisé en ce que** les molécules dudit au moins un composant libérable sont conjuguées au matériau à base de polymère par des lieurs, qui contiennent chacun la séquence peptidique dégradable par voie enzymatique.

5. Matériau à base de polymère selon la revendication 3, **caractérisé en ce que** les molécules dudit au moins un composant libérable sont incluses physiquement dans un réseau polymère tridimensionnel et/ou reliées physiquement à celui-ci.

6. Matériau à base de polymère selon l'une quelconque des revendications 1 à 5, **caractérisé en ce que** les séquences peptidiques dégradables par voie enzymatique ne sont pas dégradables par l'activité biologique et métabolique de cellules et de tissus.

7. Matériau à base de polymère selon la revendication 6, **caractérisé en ce que** la séquence peptidique clivable, qui comprend un site clivable par une protéase, est choisie dans un groupe, constitué par ENLYFQ/X, ETVLFQ/GP, EVLFQ/GP, IEGR/IEGRX, CWGGGIEGR/MGGCG, CGGGIEGR/MGGWCG, DDDDK/, LVPRGS/FXRS, DXXD/, LEVLFQ/GP, LPET/G, DX, NX, MN et SX, la barre oblique (/) indiquant à chaque fois le site clivable, les lettres représentant le code à une lettre des acides aminés protéinogènes et X représentant un acide aminé naturel quelconque.

8. Matériau à base de polymère selon la revendication 6 ou 7, comprenant en outre des séquences peptidiques qui sont dégradables par l'activité biologique et métabolique de cellules et de tissus, et sont incorporées dans le matériau à base de polymère ou conjuguées au matériau à base de polymère, de telle sorte qu'elles soient des molécules de liaison (lieurs) dans une structure bi- ou tridimensionnelle du matériau à base de polymère, de telle sorte que l'activité biologique et métabolique de cellules et de tissus permette une dégradation bio-orthogonale de la structure tridimensionnelle, ou en cas de conjugaison de la séquence peptidique au matériau à base de polymère la libération d'au moins une partie de la molécule.

9. Matériau à base de polymère selon l'une quelconque des revendications 3 à 8 pour une utilisation pour une libération *in vivo* dirigée du composant libérable, qui a lieu par une dégradation de la liaison covalente de la séquence peptidique, dirigée par ajout de la protéase.

10. Matériau à base de polymère pour l'utilisation selon la revendication 9 pour un traitement *in vivo* de cellules, de tissus et d'organes, selon lequel un agent actif et/ou un autre des composants bioactifs libérables mentionnés est libéré au besoin de manière dirigée par ajout de la protéase.

11. Utilisation d'un matériau à base de polymère selon l'une quelconque des revendications 1 à 8 pour une culture de cellules *in vitro* ou une fabrication *in vitro* de tissus ou d'organes.

12. Utilisation d'un matériau à base de polymère selon l'une quelconque des revendications 1 à 8 pour la stabilisation *in vitro* de cellules, tissus et organes donnés.

13. Utilisation d'un matériau à base de polymère selon l'une quelconque des revendications 3 à 8 pour un procédé *in vitro,* selon lequel une dégradation de la liaison covalente de la séquence peptidique, dirigée par ajout de la protéase, et ainsi une libération du composant libérable ont lieu.

14. Utilisation selon la revendication 13 lors du traitement *in vitro* de cellules, tissus ou organes vivants, dans laquelle un agent actif et/ou un autre des composants bioactifs libérables mentionnés sont libérés au besoin de manière dirigée par ajout de la protéase.


### SEQUENCE LISTING

<110> IPF Leibniz-Institut für Polymerforschung Dresden e.V.
<120> Polymerbasiertes Material mit kovalent gebundenen, enzymatisch abbaubaren Peptidsequenzen
<130> IPF17-15-EP
<140> EP 16163667.5
   <141> 2016-04-04
<160> 9
<170> PatentIn version 3.5
<210> 1
   <211> 7
   <212> PRT
   <213> Artificial Sequence
<220>
   <223> enzymatisch spaltbare Peptidsequenz, Xaa kann jede natürlich vorkommende Aminosäure sein
<220>
   <221> misc_feature
   <222> (7)..(7)
   <223> Xaa can be any naturally occurring amino acid
<400> 1
   Glu Asn Leu Tyr Phe Gln Xaa
   1               5
<210> 2
   <211> 7
   <212> PRT
   <213> Artificial Sequence
<220>
   <223> enzymatisch spaltbare Peptidsequenz
<400> 2
   Glu Val Leu Phe Gln Gly Pro
   1               5
<210> 3
   <211> 5
   <212> PRT
   <213> Artificial Sequence
<220>
   <223> enzymatisch spaltbare Peptidsequenz
<400> 3
   Asp Asp Asp Asp Lys
   1               5
<210> 4
   <211> 4
   <212> PRT
   <213> Artificial Sequence
<220>
   <223> enzymatisch spaltbare Peptidsequenz, Xaa kann jede natürlich vorkommende Aminosäure sein
<220>
   <221> misc_feature
   <222> (2)..(3)
   <223> Xaa can be any naturally occurring amino acid
<400> 4
   Asp Xaa Xaa Asp
   1
<210> 5
   <211> 9
   <212> PRT
   <213> Artificial Sequence
<220>
   <223> enzymatisch spaltbare Peptidsequenz, Xaa kann jede natürlich vorkommende Aminsosäure sein
<220>
   <221> misc_feature
   <222> (9)..(9)
   <223> Xaa can be any naturally occurring amino acid
<400> 5
   Ile Glu Gly Arg Ile Glu Gly Arg Xaa
   1               5
<210> 6
   <211> 8
   <212> PRT
   <213> Artificial Sequence
<220>
   <223> enzymatisch spaltbare Peptidsequenz
<400> 6
   Leu Glu Val Leu Phe Gln Gly Pro
   1               5
<210> 7
   <211> 5
   <212> PRT
   <213> Artificial Sequence
<220>
   <223> enzymatisch spaltbare Peptidsequenz
<400> 7
   Leu Pro Glu Thr Gly
   1               5
<210> 8
   <211> 10
   <212> PRT
   <213> Artificial Sequence
<220>
   <223> enzymatisch spaltbare Peptidsequenz, Xaa kann jede natürlich vorkommende Aminosäure sein
<220>
   <221> misc_feature
   <222> (8)..(8)
   <223> Xaa can be any naturally occurring amino acid
<400> 8
   Leu Val Pro Arg Gly Ser Phe Xaa Arg Ser
   1               5                   10
<210> 9
   <211> 7
   <212> PRT
   <213> Artificial Sequence
<220>
   <223> enzymatisch spaltbare Peptidsequenz
<400> 9
   Glu Thr Leu Phe Gln Gly Pro
   1               5